(19) Europäisches Patentamt
European Patent Office
Office européen des brevets

(11) **EP 4 442 294 A1**

(12) **EUROPEAN PATENT APPLICATION**

(43) Date of publication:
**09.10.2024 Bulletin 2024/41**

(21) Application number: **24161303.3**

(22) Date of filing: **05.03.2024**

(51) International Patent Classification (IPC):
**A61M 5/142** (2006.01)   **A61M 5/168** (2006.01)
**A61M 5/172** (2006.01)

(52) Cooperative Patent Classification (CPC):
**A61M 5/14224; A61M 5/16886; A61M 5/172;**
A61M 2205/12; A61M 2205/3334; A61M 2205/3355;
A61M 2205/3372

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **05.04.2023 US 202363494458 P**

(71) Applicant: **Fresenius Kabi USA, LLC
Lake Zurich, IL 60047 (US)**

(72) Inventors:
• **GONZALEZ, Lino
Lake Zurich, 60047 (US)**
• **SCARSELLA, Michael
Lake Zurich, 60047 (US)**
• **POWERS, Benjamin
Lake Zurich, 60047 (US)**

(74) Representative: **Maikowski & Ninnemann
Patentanwälte Partnerschaft mbB
Postfach 15 09 20
10671 Berlin (DE)**

(54) **PUMP CONTROLLER AND CONTROL METHOD WITH TEMPERATURE INTEGRATION AND/OR COMPLIANCE DETERMINATION**

(57)     A method includes calculating a volume of fluid in a pumping chamber when the chamber is not full or empty based on pressure and temperature measurements taken during a negative full-volume state, a positive full-volume state, and an empty-volume state, and an infusion system includes a controller configured to carry out such calculation. Such method, or a separate method, may measure temperature and pressure of a common volume and a pumping chamber, wherein the pressure is measured using a pressure sensor and the temperature is measured using a temperature sensor, optionally one that measures the temperature of the pressure sensor, and calculate a volume of fluid in the pumping chamber based on ideal gas law and the pressure and temperature measurements, and such infusion system, or a separate system, includes a controller configured to carry out such measurement and calculation.

FIG. 1

EP 4 442 294 A1

**Description**

BACKGROUND

This patent is directed to a pump controller that is configured to and control method that includes determination of compliance in an associated pump or pump system, and optionally is directed to such a pump controller and control method that integrates temperature measurements at various positions in the system.

Pump controllers and control methods are subject to the sensitivity of associated measuring instruments (e.g., sensors) and to factors such as compliance in the associated infusion or pump system. Compliance may be referred to as a measurement of the degree to which a component deforms under force. When referencing a pump, one may refer to the compliance of the structures of the pump as well as the compliance of the associated infusion or administration set (tubing). The compliance of the pump and the infusion set may affect the ability of the measuring instruments to provide signals that are fully representative of the conditions within the pump and/or set.

Pump controllers and control methods that rely on gas law calculations may be subject to additional factors as well. For example, the ideal gas law equation includes not only pressure and volume, but temperature as well. As such, a pump controller or control method that relies on the ideal gas law equation, but does not take into account temperature variations, may experience unaccounted for variations because of temperature fluctuations in the system.

It would be advantageous to provide a pump controller and pump control method that includes the compliance of the associated pump and/or infusion set in its determinations of fluid movement. It also would be advantageous, either separately or in combination with the preceding, to provide a pump controller and pump control method that includes temperatures within the associated pump and/or its infusion set in its determinations of fluid movement.

SUMMARY

According to one aspect of the present disclosure, a method includes applying a positive gauge pressure from a common volume to a diaphragm in a pumping chamber to empty a fluid from the pumping chamber, measuring the temperature and pressure of the common volume and the pumping chamber, wherein optionally the pressure is measured using a pressure sensor and the temperature is measured using a temperature sensor that measures the temperature of the pressure sensor, and calculating a volume of fluid in the pumping chamber based on ideal gas law and the pressure and temperature measurements.

According to another aspect of the present disclosure, an infusion system includes a common volume, a pumping chamber including a diaphragm, a fluid inlet coupled to a fluid source, and a fluid outlet, the pumping chamber coupled to the common volume, a pressure sensor and a temperature sensor associated with each of the common volume and the pumping chamber, and a controller coupled to the pressure sensors and temperature sensors. The controller is configured to apply a positive gauge pressure from a common volume to the diaphragm in the pumping chamber to empty a fluid from the pumping chamber, measure the temperature and pressure of the common volume and the pumping chamber, wherein the pressure is measured using a pressure sensor and the temperature is measured optionally using a temperature sensor that measures the temperature of the pressure sensor, and calculate a volume of fluid in the pumping chamber based on ideal gas law and the pressure and temperature measurements.

According to a further aspect of the present disclosure, a method including applying a negative gauge pressure from a common volume to a diaphragm in a pumping chamber to fill the pumping chamber with a fluid from a fluid source, applying the negative gauge pressure to the diaphragm with the pumping chamber filled with the fluid to define a negative full-volume state, and measuring temperature and pressure of the common volume and the pumping chamber during the negative full-volume state, and applying a positive gauge pressure to the diaphragm with the pumping chamber filled with the fluid to define a positive full-volume state, and measuring the temperature and pressure of the common volume and the pumping chamber during the positive full-volume state. The method also includes applying the positive gauge pressure to the diaphragm with the pumping chamber empty to define an empty-volume state, and measuring the temperature and pressure of the common volume and the pumping chamber during the empty-volume state, and calculating a volume of fluid in the pumping chamber when the chamber is not full or empty based on the pressure and temperature measurements taken during the negative full-volume state, the positive full-volume state, and the empty-volume state.

According to yet another aspect of the present disclosure, an infusion system includes a common volume, a pumping chamber including a diaphragm, a fluid inlet coupled to a fluid source, and a fluid outlet, the pumping chamber coupled to the common volume, a pressure sensor and a temperature sensor associated with each of the common volume and the pumping chamber, and a controller coupled to the pressure sensors and temperature sensors. The controller is configured to apply a negative gauge pressure from the common volume to the diaphragm in the pumping chamber to fill the pumping chamber with a fluid from the fluid source, apply the negative gauge pressure to the diaphragm with the pumping chamber filled with the fluid to define a negative full-volume state, and measure temperature and pressure of the common volume and the pumping chamber during the negative full-volume state, apply a positive gauge pressure to the diaphragm with the pumping chamber filled with the fluid to define a positive full-volume state, and measure the temperature and pressure of the common volume and the pumping chamber during the positive full-volume state, and apply the positive gauge pressure to the diaphragm with the pumping chamber empty to define an empty-volume state, and measure the temperature and pressure of the common volume and the pumping chamber during the empty-volume state. The controller is also configured to calculate a volume of fluid in the pumping chamber when the chamber is not full or empty based on the pressure and temperature measurements taken during the negative full-volume state, the positive full-volume state, and the empty-volume state.

## BRIEF DESCRIPTION OF DRAWINGS

It is believed that the disclosure will be more fully understood from the following description taken in conjunction with the accompanying drawings. Some of the figures may have been simplified by the omission of selected elements for the purpose of more clearly showing other elements. Such omissions of elements in some figures are not necessarily indicative of the presence or absence of particular elements in any of the exemplary embodiments, except as may be explicitly delineated in the corresponding written description. None of the drawings is necessarily to scale.

Fig. 1 is a schematic view of an embodiment of an infusion system with a pumping chamber.
Fig. 2 is a schematic view of an embodiment of an infusion system, including a pumping chamber as illustrated in Fig. 1 and further equipment to move air and fluid into and out of the pumping chamber.
Fig. 3 is a schematic view of the pumping chamber of Fig. 1 in a full state, with a negative gauge pressure applied to the pumping chamber.
Fig. 4 is a schematic view of the pumping chamber of Fig. 1 in a full state, with a positive gauge pressure applied to the pumping chamber.
Fig. 5 is a schematic view of the pumping chamber of Fig. 1 in an empty state.
Fig. 6 is a schematic view of a dual inlet pumping chamber showing where air can remain during an otherwise complete fill.
Fig. 7 is a schematic view of a dual inlet pumping chamber showing where air can be diverted during a pump stroke.
Fig. 8 is a flow chart of an embodiment of a method according to the present disclosure.
Fig. 9 is a flow chart of an embodiment of another method according to the present disclosure.

## DETAILED DESCRIPTION OF VARIOUS EMBODIMENTS

Infusion System Equipment and Operation, in General

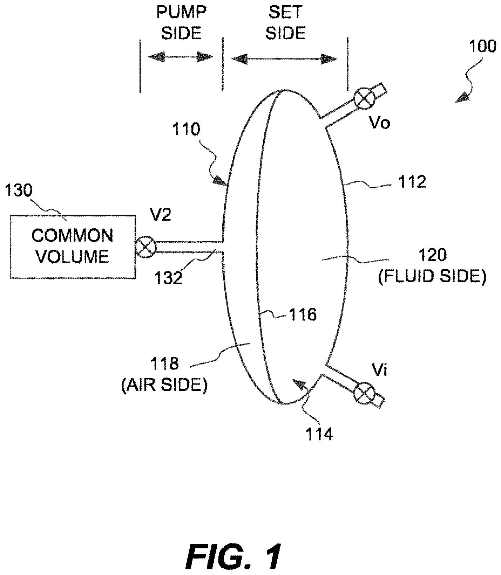

Fig. 1 is a schematic of an embodiment of an infusion, or fluid delivery, system 100. The infusion system 100 includes at least one pumping chamber 110. For reasons that will become apparent below, the pumping chamber 110 also may be referred to as the intermediate pumping chamber (IPC).

The pumping chamber 110 has a wall 112 that encloses, at least partially, a volume 114. Supported within the volume 114 is a flexible diaphragm (or membrane) 116 that divides the volume 114 into at least two spaces, first and second spaces 118, 120. According to some embodiments, the volume 114 is bifurcated by the flexible diaphragm 116. In such an embodiment, the first and second spaces 118, 120 are roughly of equal size (or volume). An example of such a chamber may be found for example, in US Pat. No. 10156231, which is incorporated by reference in its entirety herein.

The first space 118 is connected to a pumping system to move a fluid, for example a gas such as air, into and out of the first space 118. The first space 118 may be referred herein to as the air side of the chamber 110. The second space 120 is connected to a fluid source via at least a valve Vi, and to a recipient via at least a valve Vo. The fluid source may be, for example, a container of a medical fluid such as a medication or a nutritional fluid, which may also be referred to as a liquid infusate, and the recipient may be, for example, a patient or another container. The second space 120 may be referred to herein as the fluid side of the chamber 110.

The chamber 110 may be defined by structures of an administrative set, which may be disposable. The administrative set may include, for example, a cassette with the chamber 110 defined by structures of the cassette. Non-limiting examples of such a cassette may be found in US Pat. Nos. 9433734, 9849231, 9616172, 10010686, 10444770, 10881785, 11285262, which patents are incorporated by reference in their entirety herein. As such, the structures of the chamber 110 may be referred to as the set side, although it will be recognized that this is for ease of reference as the structures of the chamber should not be limited only to an embodiment where the chamber is defined by a disposable cassette of an administration set.

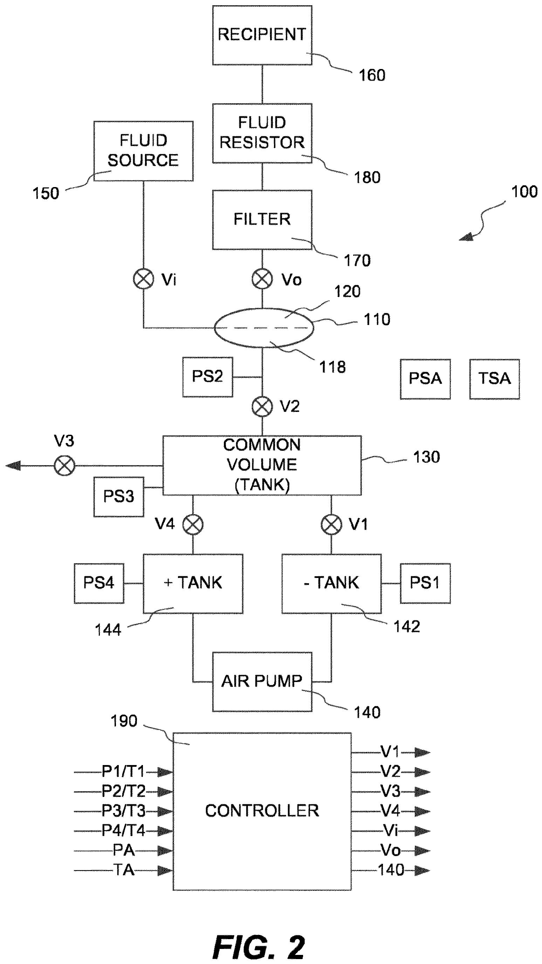

The first space 118 may be coupled (i.e., directly or indirectly connected) to reusable pumping equipment that moves the air into and out of the first space 118. Such a system is illustrated in part in Fig. 1, and will be explained in further detail in Fig. 2. The pumping equipment may include a common volume 130 (in the form of a rigid-walled tank or vessel, for example) that contains the working fluid (air) at a desired pressure. The common volume 130 is coupled to the pumping chamber 110 via a valve V2 and a port 132. In those embodiments where the pumping chamber 110 is defined as part of a disposable cassette, the cassette may have an opening that mates with the port 132 so that the air may pass into and out of the space 118. The common volume 130, valve V2, and port 132 (along with the structures connecting the valve V2 and the port 132) may be referred to as the pump side.

In a broad sense, one may refer to an air side volume to that includes a relatively fixed volume on a pump side and a variable volume on the set side (up to the diaphragm 116). Likewise, one may refer to a fluid side or liquid side volume (on the other side of the diaphragm 116). The fluid side volume varies as the medical fluid or liquid infusate is delivered or infused.

According to a method of operating the system 100, a negative gauge pressure and a positive gauge pressure are applied cyclically (via the common volume 130) to the first space 118. When a negative gauge pressure is applied to the space 118, the size of the space 120 expands and draws fluid from the fluid source into the pumping chamber 110. When a positive gauge pressure is applied to the space 118, the size of the space 120 decreases expelling fluid from the pumping chamber 110 to the recipient.

The total volume of the pumping chamber 110 is substantially constant regardless of the position of the diaphragm 116. As such, if the volume of air in the first space 118 can be determined, one is able to determine in a general sense the corresponding volume of fluid in the second space 120. For example, if the total volume is $V_{total}$ and the volume of the first space 118 is $V_{air}$, the volume of fluid in the second space 120 can be determined by subtracting $V_{air}$ from $V_{total}$. Moreover, by determining the volume of fluid in the second space 120 at different times, the delivery or infusion rate of the medical fluid may be determined.

It is desirable to improve the precision of the determination of the volume of air in the first space 118, and thereby improve the precision of the determination of the volume of fluid delivered and the determination of the delivery or infusion rate. As such, it is desirable to take into consideration the compliance of the system 100, that is how easily the components of the system 100 deform under force. For example, elastic compliance and deformation may occur because of the pressure changes in the pumping chamber 110, both on the pump side and the set side. In addition, it may be desirable to improve the precision of the determination of the volume of fluid on the fluid side by taking into consideration fluid side air bubbles that may existing the second space 120.

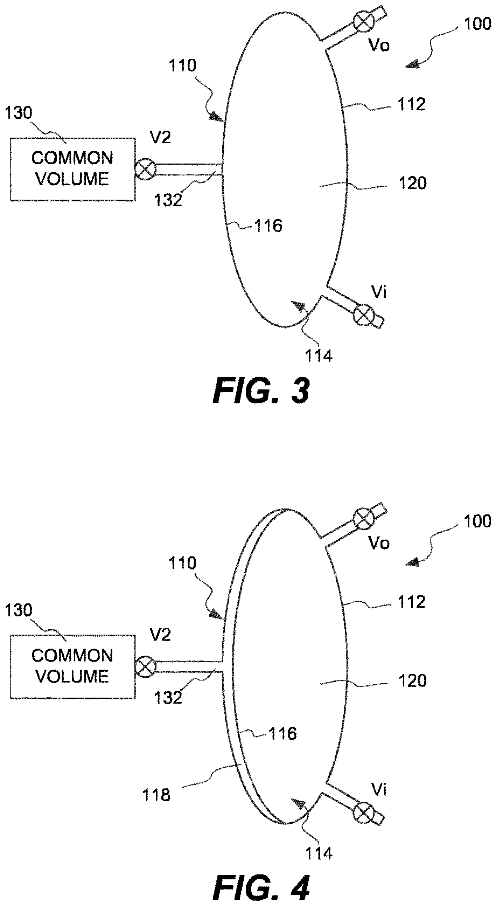

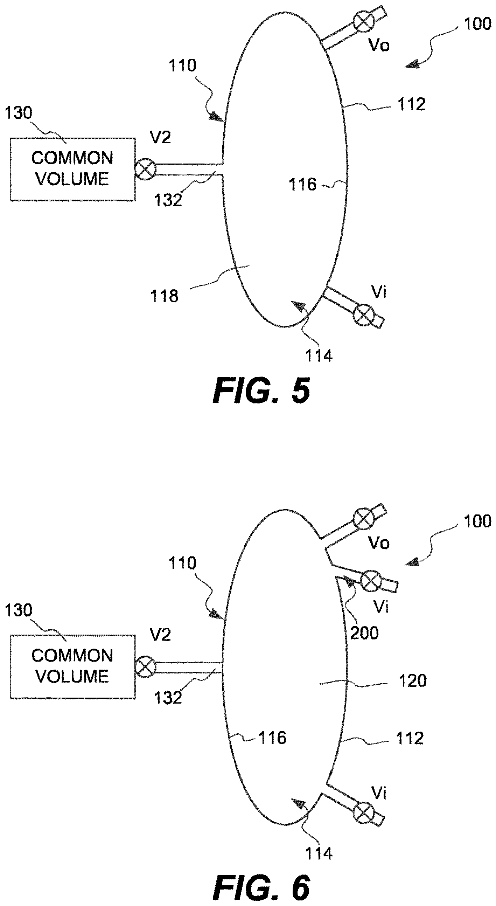

It is believed that the precision of the volume determinations may be improved by performing two different sets of measurements with the pumping chamber 110 in the full state, i.e., with the second space 120 filled with fluid, and a set of measurements taken with the pumping chamber 110 in the empty state, i.e., with the second space 120 emptied of fluid either by delivery downstream or regurgitation upstream. The former measurements (full-volume measurements) are conducted with the second space 120 filled with fluid and with a negative gauge pressure applied to the first space 118 (Fig 3), and again with a positive gauge pressure applied to the first space 118 (Fig. 4). It is believed that the first measurement is independent of set side air compliance (compliance of any air bubbles in the liquid side of the pumping chamber 110) or solid compliance (diaphragm, tubing etc. of the pumping chamber 110), but is dependent on pump side pumping chamber compliance. It is also believed that the second measurement is dependent on set side air and solid compliance, and includes a pump side volume in the first space 118. The latter measurement (empty-volume measurement) can be combined with the pump side pumping chamber volume determined in the negative full-volume measurement to determine the pumping chamber stroke volume (Fig. 5).

Once these full- and empty-volume measurements have been performed, the information may be combined with pressure and temperature measurements taken while positive gauge pressure is applied to the first space 118 to determine intermediate volumes delivered or infused. The intermediate volumes may be combined with timing information to determined flow rates, which information can be used to vary the operation of the pumping chamber to achieve a desired flow rate.

Having discussed the system 100, pumping chamber 110 and other equipment (e.g., common volume 130) in a general sense, a specific embodiment is now discussed with reference to Fig. 2. The numbering of certain structures from Fig. 1 is carried over to Fig. 2. Moreover, not all of the structures within the chamber 110 will be numbered in Fig. 2 to avoid confusion caused by the relative size of the pumping chamber 110 in Fig. 2 (as opposed to Fig. 1).

As illustrated in Fig. 2, a single pumping chamber 110 is attached to the common volume 130. According to other embodiments, more than one pumping chamber (e.g., two pumping chambers) may be attached to the common volume 130. For example, see US Pat. No. 10444770, incorporated by reference above. As such, the illustrated embodiment is provided for ease of discussion, not by way of limitation.

As mentioned above, the infusion system 100 may be operated to infuse the medical fluid (or liquid infusate) according to one or more pump cycles in accordance with a non-limiting exemplary embodiment. A pump cycle may be defined as at least partially moving the membrane 116 of the pumping chamber 110 from a first extreme (e.g., with the medical fluid expelled from the second space 120, an "empty" state) to a second extreme (e.g., with the second space 120 filled with medical fluid, a "full" state), and from the second extreme back to the first extreme. To do this, the infusion system 100 includes equipment in addition to the common volume 130 to move air into and out of the first space 118 of the pumping chamber 110.

As illustrated in Fig. 2, the infusion system 100 includes an air pump 140 that can be used to set either a positive pressure or a negative pressure in a tank. In the case of the illustrated embodiment, the air pump 140 is coupled to a first tank 142 in which a negative pressure is set, and a second tank 144 in which a positive pressure is set. When the air pump 140 is activated (turned ON) to set the tanks 142, 144 to the desired drive pressure, the valves are closed in other portions of the system 100 (in particular, valve V1 coupled between negative tank 142 and the common volume 130 and valve V4 coupled to positive tank 144 and the common volume 130).

The tanks 142, 144 are coupled to the common volume (tank) 130 via valves V1 and V4. When valve V1 is opened, the pressure in the negative tank 142 is applied to the common volume 130. When the valve V4 is opened, the pressure in the positive tank 144 is applied to the common volume 130. It is also possible to vent the common volume 130 to ambient pressure via valve V3, as may be useful at different times during a pump cycle as explained below. As mentioned above, the common volume 130 is coupled to the first space 118 of the pumping chamber 110 via the valve V2.

The infusion system 100 may include a plurality of pressure sensors disposed at various places throughout the system 100 to determine the gauge pressures of, for example, the negative tank 142 (pressure sensor PS1), the positive tank (pressure sensor PS4), the common volume 130 (pressure sensor PS3) and the first space 118 (pressure sensor PS2). According to one embodiment, each of these pressure sensors PS1 to PS4 may have a temperature sensor associated therewith, which temperature sensor provides a reading of (or measures) the temperature of the pressure sensor itself, not necessarily of the air volume to which the pressure sensor. As one example, the pressure sensors PS1 to PS4 may be Honeywell ABP pressure transducers.

In addition, the sensors PS1 to PS4 may be mounted on a single board, and a further pressure sensor PSA and a temperature sensor TSA may also be mounted thereon. The pressure sensor PSA is capable of measuring the ambient (or atmospheric) pressure, and can be used to convert the other gauge pressures into absolute pressures, which absolute pressures are used in the calculations below. The temperature sensor TSA and the sensor board may be designed to improve its ability to read the temperature outside the infusion system 100. For example, the board may be relatively thin (e.g., 0.030 inches) to reduce the thermal path from the top-side mounted sensor and the bottom of the sensor board. The electrical contacts may be designed to maximize their cross-sectional thickness. The bottom side of the board may include a thermal pad to increase surface area contact. Further, the top side of the board, including and surrounding the sensor, may be covered with an insulating foam to shield this sensor from the internal temperature of the infusion system 100.

According to one embodiment, the temperature measurement used in calculating, for example, the air side pumping chamber volume is an average air side temperature, and more particularly a mass-weighted average (although optionally other averages, such as a volume-weighted average may be used). The average, and specifically the mass-weighted average, may be calculated using temperature measurements of the pressure sensor/temperature sensors and the ambient sensor board temperature sensor. That is, while the temperature sensors do not necessarily provide the temperature of the air volume to which the pressure sensor is connected (or ported), the temperature readings provide a collection of temperatures for the pneumatic subsystem because of the physical connection of the sensors to the pneumatic manifold. In particular, the sensor board, which may be located at the base of the pump 140 near the administrative set loading mechanism and coupled to the pump housing, has been shown to provide temperature readings that closely track the set side pumping chamber temperature.

Mass-weighted average pumping chamber temperatures may be calculated according to the following equations (Eqn. 1 and 2):

$$T_{IPC,init} = \frac{V_{IPC,init}}{\dfrac{v}{T_M} + \dfrac{V_{IPC,init} - v}{T_S}}$$

$$T_{IPC,final} = \frac{V_{IPC,final}}{\dfrac{v}{T_M} + \dfrac{V_{IPC,final} - v}{T_S}}$$

where

$T_{IPC,init}$ is an initial mass-weighted average pumping chamber temperature;
$V_{IPC,init}$ is an initial pumping chamber volume;

$T_{IPC,final}$ is a final mass-weighted average pumping chamber temperature;

$V_{IPC,final}$ is a final pumping chamber volume;

v is a constant volume region within the pumping chamber;

$T_M$ is an average temperature of the plurality of temperature sensors; and

Ts is an administrative set temperature.

The equations are based on a constant volume region, v, within the pumping chamber 110 being at the average temperature of the temperature sensors (also referred to as the average pneumatic manifold temperature) $T_M$, with the remaining portion of the air side pumping chamber volume taken to be at the administration set temperature Ts.

The average pneumatic-manifold temperature $T_M$ is calculated according to the temperature measurements from the temperature sensors associated with pressure sensors PS1 to PS4. According to certain embodiments, $T_M$ may be calculated based on the temperature measurements from a subset of the temperature sensors. For example, an outlier-detection technique may be used to determine if any of the temperature measurements represents a temperature measurement that differs strongly from the other temperature measurements in the population that includes all the temperature measurements from all the temperature sensors. If no outliers are detected or if no outlier-detection technique is used, the average may be calculated, for example, as the average of the three temperature measurements closest to each other out of the four temperature measurements taken from the temperature sensors associated with pressure sensors PS1 to PS4.

The administrative set temperature Ts is calculated based on $T_M$ and $T_A$, which is the temperature measurement of ambient temperature sensor TSA. In particular, according to certain embodiments, Ts is an interpolated value between $T_A$ and $T_M$. For example, the calculation may be performed according to the following equation (Eqn. 3):

$$T_S = T_A + \beta(T_M - T_A)$$

where β is a parameter. During an initialization, the set side of the pumping chamber will generally be close to room temperature. Accordingly, β may be set to zero at such times. After initialization, β may be set to a non-zero positive value (e.g., 0.125) to account for pre-heating of the set side pumping chamber volume by the warmer pump side pumping chamber air.

The second space 120 of the pumping chamber 110 is coupled to a fluid source (e.g., a flexible bag or rigid/semi-rigid walled container) 150 via the valve Vi. The second space 120 of the pumping chamber is also coupled to a recipient 160 (e.g., a patient or a container) via the valve Vo. As illustrated, the recipient 160 may be coupled to the pumping chamber 110 via a filter 170 and a fluid resistor 180, such as an adjustable fluid resistor (for example, an incrementally adjustable fluid resistor) that may be configured to vary the in-line fluid resistance. According to other embodiments, further equipment may also be coupled between the pumping chamber 110 and the recipient 160.

To control all of the valves Vi, Vo, and V1 to V4 and the air pump 140, a controller 190 is provided that is coupled to the valves Vi, Vo, and V1 to V4 and the air pump 140. The controller 190 may also be coupled to the fluid resistor 180 to vary the in-line fluid resistance. In addition, the controller 190 may also be coupled to the pressure sensors PS1 to PS4 and PSA and temperature sensor TSA, and may receive signals therefrom representative of pressure (P1 to P4 and PA) and of temperature (T1 to T4 and TA). Furthermore, the controller 190 may be coupled to additional equipment, including input devices (e.g., touch screens, keyboards, buttons, etc.) and/or output devices (e.g., electronic display devices, light emitting diodes, audio output devices such as speakers, etc.).

The controller 190 may include for example a processor and memory (or storage device), electrical circuits, or a combination of a processor and memory and electrical circuits. The controller 190 may be configured according to any of the methods of operating the system 100 discussed herein. Where the controller 190 includes a processor and memory, the controller 190 may be programmed to perform any of the methods of operating the system 100 discussed herein, and the memory may include a non-transitory computer readable medium on which the instructions executed by the controller 190 are stored.

In particular, the controller 190 may be configured to carry out one or more of the following non-limiting exemplary embodiments of a method for operating the infusion system 100.

To perform a pump cycle, the controller 190 sets the valves V1 to V4 to a closed state or position. The controller 190 then activates the air pump 140 (sets air pump 140 to the ON state) to bring the positive and negative tanks 142, 144 to the desired drive pressures.

Next, the controller 190 sets valves V1 and V2 to an open state or position to apply the pressure in negative tank 142 to the pumping chamber 110. The negative gauge pressure draws the diaphragm 116 towards the pump side, allowing the second space 120 to fill with medical fluid from the fluid source 150. With the second space 120 filled with medical fluid, the controller 190 sets valve V1 to the closed state, and sets valves V2 and V4 to an open state or position to apply the pressure in the positive tank 144 to the pumping chamber 110. The positive gauge pressure pushes the diaphragm 116 towards the set side, expelling the medical fluid from the second space 120 to flow towards the recipient.

At this point, the controller 190 may reset the pressures in the tanks 142, 144, and repeat the pump cycle.

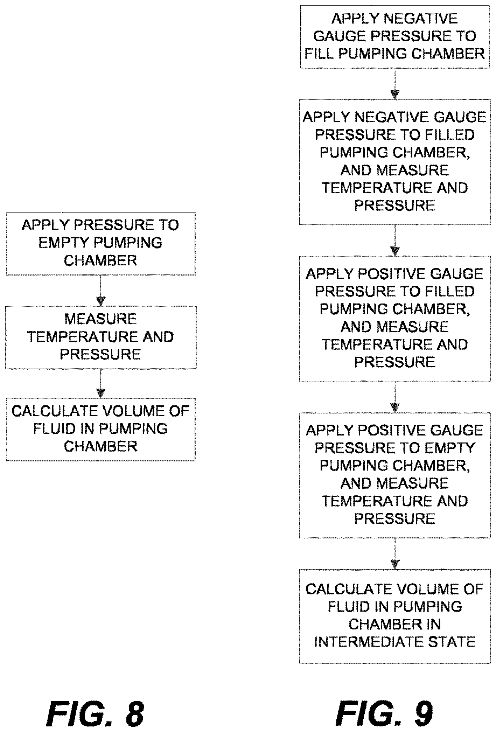

In addition to performing one or more pump cycles, the controller 190 may perform volume calculations at one or more times after the first space 118 of the pumping chamber 110 is brought to positive gauge pressure. See Fig. 8, for example. These volume calculations may be performed periodically during the time that the pumping chamber 110 is emptying (e.g., as the medical fluid is being expelled from the second space 120). As these volume calculations are being performed, a flow rate also may be calculated based on the volume calculations and the times the volume calculations are performed.

Based on the calculated flow rate, the controller may adjust the one or both of the target drive pressure in the positive tank 144 and the in-line resistance as determined by the fluid resistor 180. In general terms, increasing the pressure of the gas in the space 118 of the pumping chamber 110 increases the rate of fluid delivery, and decreasing the pressure decreases the rate of fluid delivery. On the other hand, increasing the in-line fluid resistance decreases the rate of fluid delivery to the recipient 160, while decreasing the in-line fluid resistance increases the rate of fluid delivery to the recipient 160.

Volume Calculations, in Specific

In addition to performing one or more pump cycles and performing volume and flow rate calculations, the controller 190 may perform additional calculations to compensate for compliance on pump side and set side, as well as for air bubbles present on the fluid side of the pumping chamber 110. These calculations may be advantageously improved by the use of the pressure sensors with associated temperature sensors described above.

Returning to Fig. 1, the pumping chamber 110 includes several volumes, or volume-segments. As illustrated and described above, the pumping chamber 110 may include equipment on a pump side of the infusion system 100, and equipment on a mating administration set side of the infusion system 100. When the administration set is mated with the pump, an air-side pumping chamber volume may include a fixed volume segment on the pump side, and a variable volume segment on the set side. The air side/set side volume segment varies according to the amount of liquid infusate in the fluid side of the pumping chamber.

As discussed above, by measuring the air side volume of the pumping chamber the fluid side volume may be determined, and by measuring the air side volume of the pumping chamber at multiple times the infusate flow out of the pump may be determined. That is, the change in air side pumping chamber volume may be used to calculate the amount of liquid delivered between the measurements, and thus the infusate flow.

In a general sense, the air side volume measurements are performed in the following manner. To begin, the pumping chamber 110 and the common volume 130 are at different pressures. Next, the pumping chamber 110 and the common volume 130 are combined by opening valve V2, with all other valves (V1, V3, V4) closed. Then, the pumping chamber air side volume is calculated using pressure and temperatures measurements, taking into account conservation of mass, the ideal gas law, and the known, calibrated reference volume of the common volume 130 (which serves as a reference point). The temperatures may be determined according to Equations 1 and 2, above.

As stated above, once the air side volume is known, the amount of liquid delivered between measurements can be calculated. According to the present disclosure, the calculation of the amount of liquid delivered is performed after compensating for compliance in the system. In particular, the compensation addresses liquid side air bubbles, as well as elastic compliance and deformation caused by pressure changes in the pumping chamber 110, both on the pump and set sides.

To compensate, additional measurements are taken with the pumping chamber in a full state and in an empty state. See, for example, Fig. 9. The measurements in the full state are taken both where the pumping chamber has a negative gauge pressure during the measurement sequence and a positive gauge pressure during the measurement sequence. See Figs. 3 and 4. The difference between the negative full volume and positive full volume measurements is used to calculate the amount of air present on the liquid side of the pumping chamber 110. The negative and positive full volume measurements may be taken, for example, sequentially after each fill of the pumping chamber 110. The measurements in the empty state are taken where the pumping chamber has a positive gauge pressure. See Fig. 5. The measurements are used to determine the total pumping chamber volume (both on air and fluid sides), and to calculate the pumping chamber stroke volume.

Full volume measurements

As noted above, the combination of the negative full volume and the positive full volume measurements permit the volume of air on the fluid or liquid side of the pumping chamber 110 to be calculated. Each of the measurements, negative and positive, calculates a particular volume, and may be independent of certain compliances while dependent on other compliances. Thus, before continuing, we discuss each of the full volume measurements separately.

The negative full volume measurements are used to calculate the pump side pumping chamber volume. During the negative full volume measurements, the air side of the pumping chamber is at a lower pressure than the hydrostatic fluid pressure throughout the measurement sequence. Consequently, after a complete fill, the diaphragm 116 will rest against a portion of the wall 112 of the pumping chamber 110 closest to the common volume 130 (the near wall). See Fig. 3. This condition effectively decouples the pump side and set side portions of the pumping chamber 110 from each other. As a further consequence, the pump side pressure of the pumping chamber 110 is equal to the pressure measured by pressure sensor PS2. The set side of the pumping chamber remains at the hydrostatic inlet pressure of the fluid. Negative full volume measurements are thus independent of any air or solid compliance present in the set at the time of the measurement, but dependent on pump side pumping chamber compliance.

The positive full volume measurements are used to calculate the total volume of air in the pumping chamber (air side and fluid side). During the positive full volume measurements, the air side of the pumping chamber 110 is at a higher pressure than the hydrostatic fluid pressure through the measurement sequence. Consequently, the diaphragm will be pushed off the near wall. See Fig. 4. This equilibrates the different pumping chamber volumes. As a further consequence, the pump side and both set side (air and fluid) pressures will be equal to the value measured by the pressure sensor PS2. With the inlet and outlet valves Vi and Vo closed, the positive full volume measurements will measure the air side pumping chamber volume, and be dependent on the compliance of any air bubbles/pockets in the liquid side of the pumping chamber and the solid compliance.

Like the other measurements made herein, the negative full volume and positive full volume measurements are performed in following manner. To begin, the pumping chamber 110 and the common volume 130 are at different pressures. A first, or initial, set of pressure and temperature measurements are taken. Next, the pumping chamber 110 and the common volume 130 are combined by opening valve V2, with all other valves (V1, V3, V4) closed. A second, or final, set of pressure and temperature measurements are taken. The two sets of measurements are then used to calculate the volume.

So, with respect to the negative full volume measurements, the controller 190 brings the pumping chamber 110 to a target pressure of around -6 psig. The valve V2 is then closed, and the common volume 130 is vented to ambient pressure by opening V3. After valve V3 is closed, an initial set of pressure and temperature measurements are taken in the pumping chamber 110 (PS2) and the common volume 130 (PS3) and by the ambient sensors (PSA, TSA). Then the valve V2 is opened, and the two air volumes (pumping chamber 110, common volume 130) are merged and allowed to equilibrate. A final set of pressure and temperature measurements are taken again in each chamber and by the ambient sensors.

Similarly, with respect to the positive full volume measurements, the controller 190 brings the pumping chamber 110 to the target drive pressure. The valve V2 is the closed, and the common volume 130 is vented to ambient pressure by opening V3. After valve V3 is closed, an initial set of pressure and temperature measurements are taken in the pumping chamber 110 (PS2) and the common volume 130 (PS3) and by the ambient sensors (PSA, TSA). Then the valve V2 is opened, and the two air volumes (pumping chamber 110, common volume 130) are merged and allowed to equilibrate. A final set of pressure and temperature measurements are taken again in each chamber and by the ambient sensors.

In the same way that a similar method is used for taking the measurements, the equations used to determine the desire volumes are based on conversation of air mass, the ideal gas law, and the temperature equations (Eqn. 1 and 2) above. A negative sign (-) is used in subscripts to denote negative full-volume measurements, and a positive sign (+) is used in subscripts to denote positive full-volume measurements.

Starting with the negative full volume measurements, the sum of moles of air in the volumes (pumping chamber 110 and common volume 130) is the same before and after the volumes are merged, and may be represented by the following equation (Eqn. 4):

$$n_{IPC,init,-} + n_{COM,init,-} = n_{IPC,final,-} + n_{COM\,final,-}$$

where

$n_{IPC,init,-}$ is moles of air in the pumping chamber volume initially;
$n_{IPC,final,-}$ is moles of air in the pumping chamber volume finally;
$n_{COM,init,-}$ is moles of air in the common volume initially; and
$n_{COM,final,-}$ is moles of air in the common volume finally.

Additionally, the ideal gas law may be used to relate the pressure, temperature, and volume before and after the volumes are merged, as expressed in the following equation (Eqn. 5):

$$\frac{P_{IPC,init,-}V_{IPC,init,-}}{T_{IPC,init,-}} + \frac{P_{COM,init,-}V_{COM}}{T_{COM,-}} = \frac{P_{IPC,final,-}V_{IPC,final,-}}{T_{IPC,final,-}} + \frac{P_{COM,final,-}V_{COM}}{T_{COM,-}}$$

where

$P_{IPC,init,-}$, $V_{IPC,init,-}$ and $T_{IPC,init,-}$ are the pressure, volume, and temperature in the pumping chamber volume initially;
$P_{IPC,final,-}$, $V_{IPC,final,-}$ and $T_{IPC,final,-}$ are the pressure, volume, and temperature in the pumping chamber volume finally;
$P_{COM,init,-}$ and $P_{COM,final,-}$ are the pressure in the common volume initially and finally; and
$V_{COM}$ and $T_{COM}$ are the volume and temperature in the common volume.

As reflected in Eqn. 5, the volume of the common volume is the same both initially and finally (because of its high stiffness) and the temperature is the same both initially and finally. It is believed that the pump side pumping chamber volume, on the other hand, will vary with measurement pressure because of compliance of the passage (e.g., tubing material). Similarly, the mass-weighted temperature used for the pumping chamber (see Eqn. 1 and 2, above) can vary with pressure because of the compliance of the passage. All pressure and temperature terms are absolute.

It will be recognized that the nominal (unpressurized) pump side pumping chamber volume is equal to the volume of the measurement pressure less the volume change caused by membrane compliance. The volume may be expressed either in terms of the initial or final measurement volumes and pressures as follows (Eqn. 6):

$$V_{IPC,pump} = V_{IPC,init,-} + k_{pump}\left(P_A - P_{IPC,init,-}\right)$$

$$= V_{IPC,final,-} + k_{pump}\left(P_A - P_{IPC,final,-}\right)$$

where
$k_{pump}$ is the effective compliance on the pump side of the pumping chamber volume (ratio of volume change to pressure change during the measurement sequence, e.g., 0.0007 cc/psi).

Combining the foregoing equations, the nominal (unpressurized) pump side pumping chamber volume is obtained from the following equation, which combines the previous expressions and takes into account membrane compliance (Eqn. 7):

$$V_{IPC,pump} = V_{COM}r_{PN} + k_{pump}\left(P_A - P_{IPC,init,-} - P_{IPC,final,-}\right)$$

where
$r_{PN}$ is a temperature-corrected pressure ratio, calculated according to the following equation (Eqn. 8):

$$r_{PN} \stackrel{\text{def}}{=} \tilde{r}_{PN}\frac{T_{S,-}}{T_{M,-}} + \frac{v}{V_{COM}}\left(1 - \frac{T_{S,-}}{T_{M,-}}\right)$$

where
$\tilde{r}_{PN}$ is a dimensionless pressure ratio, calculated according to the following equation (Eqn. 9):

$$\tilde{r}_{PN} \stackrel{\text{def}}{=} \frac{P_{COM,final,-} - P_{COM,init,-}}{P_{IPC,init,-} - P_{IPC,final,-}}$$

The dimensionless pressure ratio and the temperature-corrected pressure ratio implicitly assume that negative gauge pressures lower than hydrostatic liquid pressure are used for the initial and final pumping chamber temperatures. To minimize the effect of thermal transients during venting and merging of the air volumes, the system is allowed to equilibrate before air pressures are recorded.

Having discussed the negative full volume calculations, it will be recognized that much of the foregoing can be used in regard to the positive full volume calculations, as far as the conservation of air mass and ideal gas law equations would be the same as Equations 4 and 5, above, except that the negative signs (-) would be replaced with positive signs (+). Further, It will be recognized that the nominal (unpressurized) air volume in the pumping chamber is equal to the volume of the measurement pressure less the volume change caused by membrane compliance. The volume may be expressed either in terms of the initial or final measurement volumes and pressures as follows (Eqn. 10):

$$V_{IPC,pump} + V_{air} = V_{IPC,init,+} - \left(k_{pump} + k_{set}\right)\left(P_{IPC,init,\pm} - P_A\right) - \sigma$$

$$= V_{IPC,final,+} - \left(k_{pump} + k_{set}\right)\left(P_{IPC,final,+} - P_A\right) - \sigma$$

where

$k_{set}$ is the effective compliance on the set side of the pumping chamber volume (ratio of volume change to pressure change during the measurement sequence, e.g., 0.0013 cc/psi); and
$\sigma$ is a volume measurement offset that occurs when the pumping chamber is pressurized above ambient pressure, causing the collapsed volume around the fluid inlet and outlet valves Vi and Vo to become filled with fluid.

Combining the foregoing equations, the total amount of air in the pumping chamber (air side and fluid side) is obtained from the following equation, which combines the previous expressions and takes into account membrane compliance (Eqn. 11):

$$V_{air} = V_{COM}\left(r_{PP} - r_{PN}\right) - k_{set}\left(P_{IPC,init,+} + P_{IPC,final,+} - P_A\right)$$

$$- k_{pump}\left(P_{IPC,init,+} + P_{IPC,final,+} - P_{IPC,init,-} - P_{IPC,final,-}\right) - \sigma$$

where
$r_{PP}$ is a temperature-corrected pressure ratio, calculated according to the following equation (Eqn. 12):

$$r_{PP} \overset{\text{def}}{=} \tilde{r}_{PP}\frac{T_{S,+}}{T_{M,+}} + \frac{v}{V_{COM}}\left(1 - \frac{T_{S,+}}{T_{M,+}}\right)$$

where
$\tilde{r}_{PP}$ is a dimensionless pressure ratio, calculated according to the following equation (Eqn. 13):

$$\tilde{r}_{PP} \overset{\text{def}}{=} \frac{P_{COM,final,+} - P_{COM,init,+}}{P_{IPC,init,+} - P_{IPC,final,+}}$$

The dimensionless pressure ratio and the temperature-corrected pressure ratio implicitly assume that positive gauge pressures higher than hydrostatic liquid pressure are used for the initial and final pumping chamber temperatures. As above, to minimize the effect of thermal transients during venting and merging of the air volumes, the system is allowed to equilibrate before air pressures are recorded.

Empty volume measurements

As noted above, empty volume measurements are used to determine the total pumping chamber volume (both on air and fluid sides). The total pumping chamber volume can be converted to a pumping chamber stroke volume, corresponding to the fluid delivered during a full pumping chamber stroke, by taking the difference of this volume and the volume calculated according to the negative full volume measurement.
Like the other measurements made herein, and most like the positive full volume measurements, the empty volume measurements are performed in following general manner. To begin, the pumping chamber 110 and the common volume 130 are at different pressures. A first, or initial, set of pressure and temperature measurements are taken. Next, the pumping chamber 110 and the common volume 130 are combined by opening valve V2, with all other valves (V1, V3, V4) closed. A second, or final, set of pressure and temperature measurements are taken. The two sets of measurements are then used to calculate the volume.

In particular, the controller 190 brings the pumping chamber 110 to the target drive pressure. The valve V2 is the closed, and the common volume 130 is vented to ambient pressure by opening V3. After valve V3 is closed, an initial set of pressure and temperature measurements are taken in the pumping chamber 110 (PS2) and the common volume 130 (PS3) and by the ambient sensors (PSA, TSA). Then the valve V2 is opened, and the two air volumes (pumping chamber 110, common volume 130) are merged and allowed to equilibrate. A final set of pressure and temperature measurements are taken again in each chamber and by the ambient sensors.

As for calculations, the conservation of mass, the application of the ideal gas law, and the calculation of mass-weighted average temperatures are the same as for the full volume measurements above. As such the total nominal pumping chamber volume may be calculated as follows (Eqn. 14):

$$V_{IPC} = V_{COM}r_{PP} - k_{set}\left(P_{IPC,init,+} + P_{IPC,final,+} - P_A\right)$$

$$- k_{pump}\left(P_{IPC,init,+} + P_{IPC,final,+} - P_A\right)$$

Moreover, the total nominal (unpressurized) pumping chamber stroke volume may be calculated by taking the difference between the total nominal pumping chamber volume and the nominal pump side pumping chamber volume (Eqn. 15):

$$V_{IPC,stroke} = V_{COM}\left(r_{PP} - r_{PN}\right) - k_{set}\left(P_{IPC,init,+} + P_{IPC,final,+} - P_A\right)$$

$$- k_{pump}\left(P_{IPC,init,+} + P_{IPC,final,+} - P_{IPC,init,-} - P_{IPC,final,-}\right) - \sigma$$

The terms with the positive sign (+) and the pressure ratio $r_{PP}$ correspond to the positive empty volume measurements. The terms with the negative sign (-) and the pressure ratio $r_{PN}$ refer to the negative full volume measurements, above.

The stroke volume may be periodically updated during an infusion, or may be determined once during the initialization of the system 100. For example, during an infusion with a high flow, the stroke volume may be periodically updated. During an infusion with a low flow rate, the stroke volume may be determined during system initialization, and may not be updated until the infusion system 100 is rebooted.

The amount of liquid that can be delivered during a pumping chamber stroke $V_{IPC,fluid}$ is calculated according to the following calculation (Eqn. 16):

$$V_{IPC,fluid} = V_{IPC,stroke} - V_{air}$$

Intermediate volume measurements

With this information, the controller 190 can calculate the amount of volume delivered from the start of the pump stroke according to the following equation (Eqn. 17):

$$V_{IPC,partial} = V_{COM}\left(r_{PP} - r_{PN}\right) - k_{set}\left(P_{IPC,init,+} + P_{IPC,final,+} - P_A\right)$$

$$- k_{pump}\left(P_{IPC,init,+} + P_{IPC,final,+} - P_{IPC,init,-} - P_{IPC,final,-}\right) - \sigma - V_{air}$$

In addition, the amount of liquid delivered during a pump stroke between times, $t_1$ and $t_2$ may be calculated according to the following equation (Eqn. 18):

$$\Delta V_{IPC,partial} = V_{IPC,partial}(t_2) - V_{IPC,partial}(t_1)$$

where $\Delta V_{IPC,partial}$ is the amount of liquid delivered (or change in volumes);

$V_{IPC,partial}(t_2)$ is the volume at time $t_2$; and
$V_{IPC,partial}(t_1)$ is the volume at time $t_1$.

Dual inlet variant

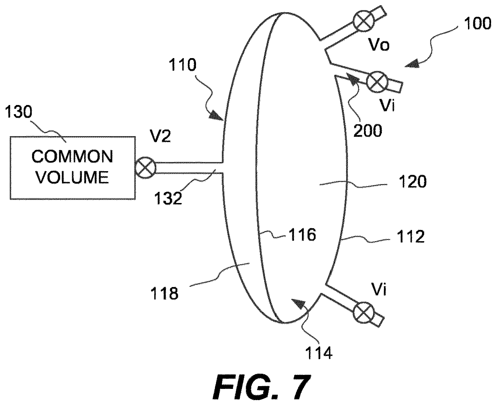

Figs 6 and 7 show a variant of the pumping chamber wherein the pumping chamber is connected to two fluid sources by two inlets (dual inlet). In this variant, air can remain trapped on the fluid side of the pumping chamber after the set is primed. As illustrated in Fig. 6 by reference numeral 200, air can be trapped in a secondary inlet if only the primary inlet is primed before the start of the infusion. As illustrated in Fig. 7, during subsequent fills of the pumping chamber 110, a fraction of the trapped air may act as an excluded volume (again reference numeral 200), preventing the pumping chamber 110 from filling completely with fluid. During delivery, the trapped air volume is compressed by the drive pressure, resulting in a portion of the fluid in the space 120 being diverted into the secondary inlet to fill space previously occupied by the now compressed air. As such, the fluid diverted into the secondary inlet is not delivered to the outlet.

To reflect this in the foregoing the equations, the volume Vair may be replaced by an effective air volume $V_{air,eff}$ according to the following (Eqn. 19):

$$V_{air,eff} = \gamma V_{air} + (1 - \gamma)V_{air} \frac{P_A}{P_{IPC,drive}}$$

where $\gamma$ is a volume fraction, e.g., 0.5; and
$P_{IPC,drive}$ is the drive pressure being used to deliver fluid.

The effective volume may be used in the foregoing equations when calculating the amount of fluid delivered to the patient during full or partial strokes of the pumping chamber 110. For example, the equation for determining the air in the pumping chamber 110 in a partial stroke may be as follows (Eqn. 20):

$$V_{IPC,partial} = V_{COM}(r_{PP} - r_{PN}) - k_{set}(P_{IPC,init,+} + P_{IPC,final,+} - P_A)$$

$$- k_{pump}(P_{IPC,init,+} + P_{IPC,final,+} - P_{IPC,init,-} - P_{IPC,final,-}) - \sigma$$

$$- V_{air,eff}$$

Similarly, the amount of liquid that can be delivered during a pumping chamber stroke $V_{IPC,fluid}$ may be calculated according to the following calculation (Eqn. 21):

$$V_{IPC,fluid} = V_{IPC,stroke} - V_{air,eff}$$

Moreover, because $V_{air,eff}$ is calculated after every pumping chamber fill, the active version of $V_{IPC,fluid}$ is updated after every pumping chamber fill even if $V_{IPC,stroke}$ is not.

Although the preceding text sets forth a detailed description of different embodiments of the invention, it should be understood that the legal scope of the invention is defined by the words of the claims set forth at the end of this patent. The detailed description is to be construed as exemplary only and does not describe every possible embodiment of the invention since describing every possible embodiment would be impractical, if not impossible. Numerous alternative embodiments could be implemented, using either current technology or technology developed after the filing date of this patent, which would still fall within the scope of the claims defining the invention.

Aspects

Aspect 1. A method comprising:

applying a positive gauge pressure from a common volume to a diaphragm in a pumping chamber to empty a fluid from the pumping chamber;
measuring the temperature and pressure of the common volume and the pumping chamber; and

calculating a volume of fluid in the pumping chamber based on ideal gas law and the pressure and temperature measurements.

Aspect 2. The method according to Aspect 1, wherein the pressure is measured using a pressure sensor and the temperature is measured using a temperature sensor that measures the temperature of the pressure sensor.

Aspect 3. The method according to Aspect 1 or 2, wherein the temperature measurements are based on an average of temperature measurements from a plurality of temperature sensors and an ambient temperature.

Aspect 4. The method according to Aspect 3, where the plurality of temperature sensors are associated with the common volume and the pumping chamber, and a positive pressure tank and a negative pressure tank coupled to the common volume.

Aspect 5. The method according to Aspect 3 or 4, wherein the temperature measurements are calculated as mass-weighted temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature.

Aspect 6. The method according to Aspect 5, wherein the administrative set temperature is calculated according to:

$$T_S = T_B + \beta(T_M - T_B),$$

where Ts is the administrative set temperature;

$T_A$ is the ambient temperature;

$\beta$ is a parameter that accounts for pre-heating of the set side pumping chamber volume by pump side pumping chamber air; and

$T_M$ is the average of temperature measurements of the plurality of temperature sensors.

Aspect 7. The method according to any one of Aspects 3 to 6, wherein the average of temperature measurements of the plurality of temperature sensors is taken of temperature measurements of a subset of the plurality of temperature sensors.

Aspect 8. An infusion system comprising:

a common volume;

a pumping chamber comprising a diaphragm, a fluid inlet coupled to a fluid source, and a fluid outlet, the pumping chamber coupled to the common volume;

a pressure sensor and a temperature sensor associated with each of the common volume and the pumping chamber;

a controller coupled to the pressure sensors and temperature sensors, the controller configured to:

apply a positive gauge pressure from a common volume to a diaphragm in the pumping chamber to empty a fluid from the pumping chamber;

measure the temperature and pressure of the common volume and the pumping chamber; and

calculate a volume of fluid in the pumping chamber based on ideal gas law and the pressure and temperature measurements.

Aspect 9. The system according to Aspect 8, wherein the pressure is measured using a pressure sensor and the temperature is measured using a temperature sensor that measures the temperature of the pressure sensor.

Aspect 10. The system according to Aspect 8 or 9, wherein the temperature measurements are based on an average of temperature measurements from a plurality of temperature sensors and an ambient temperature.

Aspect 11. The system according to Aspect 10, wherein the plurality of temperature sensors are associated with the common volume and the pumping chamber, and a positive pressure tank and a negative pressure tank coupled to the common volume.

Aspect 12. The system according to Aspect 10 or 11, wherein the controller is configured to calculate the temperature measurements as mass-weighted average temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature.

Aspect 13. The system according to Aspect 12, wherein the controller is configured to calculate the administrative set temperature according to:

$$T_S = T_B + \beta(T_M - T_B),$$

where Ts is the administrative set temperature;
$T_A$ is the ambient temperature;
$\beta$ is a parameter that accounts for pre-heating of the set side pumping chamber volume by pump side pumping chamber air; and
$T_M$ is the average of temperature measurements of the plurality of temperature sensors.

Aspect 14. The system according to any one of Aspects 10 to 13, wherein the average of temperature measurements of the plurality of temperature sensors is taken of temperature measurements of a subset of the plurality of temperature sensors.

Aspect 15. A method comprising:

applying a negative gauge pressure from a common volume to a diaphragm in a pumping chamber to fill the pumping chamber with a fluid from a fluid source;
applying the negative gauge pressure to the diaphragm with the pumping chamber filled with the fluid to define a negative full-volume state, and measuring temperature and pressure of the common volume and the pumping chamber during the negative full-volume state;
applying a positive gauge pressure to the diaphragm with the pumping chamber filled with the fluid to define a positive full-volume state, and measuring the temperature and pressure of the common volume and the pumping chamber during the positive full-volume state;
applying the positive gauge pressure to the diaphragm with the pumping chamber empty to define an empty-volume state, and measuring the temperature and pressure of the common volume and the pumping chamber during the empty-volume state; and
calculating a volume of fluid in the pumping chamber when the chamber is not full or empty based on the pressure and temperature measurements taken during the negative full-volume state, the positive full-volume state, and the empty-volume state.

Aspect 16. The method according to Aspect 15, further comprising calculating a pump side pumping chamber volume based on the pressure and temperature measurements taken during the negative full-volume state.

Aspect 17. The method according to Aspect 15 or 16, further comprising calculating a total volume of air in the pumping chamber on air side and fluid side based on the pressure and temperature measurements taken during the positive full volume state.

Aspect 18. The method according to Aspect 16 and 17, further comprising calculating a difference between the pump side pumping chamber volume and the total volume of air in the pumping chamber to calculate a volume of air on the fluid side of the pumping chamber.

Aspect 19. The method according to any one of Aspects 15 to 18, further comprising calculating a total pumping chamber volume based on the pressure and temperature measurements taken during the empty volume state.

Aspect 20. The method according to Aspect 16 and 19, further comprising calculating a stroke volume according to the difference between the total pumping chamber volume and the pump side pumping chamber volume.

Aspect 21. The method according to any one of Aspects 15 to 20, wherein the temperature measurements are based on an average of temperature measurements from a plurality of temperature sensors and an ambient temperature.

Aspect 22. The method according to Aspect 21, wherein the plurality of temperature sensors are associated with the common volume and the pumping chamber, and a positive pressure tank and a negative pressure tank coupled to the common volume.

Aspect 23. The method according to Aspect 21 or 22, wherein the temperature measurements are calculated as mass-weighted average temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature.

Aspect 24. The method according to Aspect 23, wherein the administrative set temperature is calculated according to:

$$T_S = T_B + \beta(T_M - T_B),$$

where Ts is the administrative set temperature;

$T_A$ is the ambient temperature;

β is a parameter that accounts for pre-heating of the set side pumping chamber volume by pump side pumping chamber air; and

$T_M$ is the average of temperature measurements of the plurality of temperature sensors.

Aspect 25. The method according to any one of Aspects 21 to 24, wherein the average of temperature measurements of the plurality of temperature sensors is taken of temperature measurements of a subset of the plurality of temperature sensors.

Aspect 26. The method according to any one of Aspects 21 to 25, wherein each of the plurality of temperature sensors comprise a temperature sensor associated with a pressure sensor.

Aspect 27. The method according to any one of Aspects 15 to 20, further comprising isolating the filled pumping chamber from the fluid source and a recipient before applying the negative gauge pressure to the diaphragm with the pumping chamber filled with the fluid, and before applying the positive gauge pressure to the diaphragm with the pumping chamber filled with the fluid.

Aspect 28. The method according to Aspect 27, an inlet valve disposed between the pumping chamber and the fluid source and an outlet valve disposed between the pumping chamber and the recipient, wherein isolating the pumping chamber comprises closing the inlet valve and the outlet valve.

Aspect 29. An infusion system comprising:

a common volume;

a pumping chamber comprising a diaphragm, a fluid inlet coupled to a fluid source, and a fluid outlet, the pumping chamber coupled to the common volume;

a pressure sensor and a temperature sensor associated with each of the common volume and the pumping chamber;

a controller coupled to the pressure sensors and temperature sensors, the controller configured to:

apply a negative gauge pressure from the common volume to the diaphragm in the pumping chamber to fill the pumping chamber with a fluid from the fluid source;

apply the negative gauge pressure to the diaphragm with the pumping chamber filled with the fluid to define a negative full-volume state, and measure temperature and pressure of the common volume and the pumping chamber during the negative full-volume state;

apply a positive gauge pressure to the diaphragm with the pumping chamber filled with the fluid to define a positive full-volume state, and measure the temperature and pressure of the common volume and the pumping chamber during the positive full-volume state;

apply the positive gauge pressure to the diaphragm with the pumping chamber empty to define an empty-volume state, and measure the temperature and pressure of the common volume and the pumping chamber during the empty-volume state; and

calculate a volume of fluid in the pumping chamber when the chamber is not full or empty based on the pressure and temperature measurements taken during the negative full-volume state, the positive full-volume state, and the empty-volume state.

Aspect 30. The system according to Aspect 29, wherein the controller is configured to calculate a pump side pumping chamber volume based on the pressure and temperature measurements taken during the negative full-volume state.

Aspect 31. The system according to Aspect 29 or 30, wherein the controller is configured to calculate a total amount of air in the pumping chamber on air side and fluid side based on the pressure and temperature measurements taken during the positive full volume state.

Aspect 32. The system according to Aspects 30 and 31, wherein the controller is configured to calculate a difference between the pump side pumping chamber volume and the total volume of air in the pumping chamber to calculate a volume of air on the fluid side of the pumping chamber.

Aspect 33. The system according to any one of Aspects 29 to 32, wherein the controller is configured to calculate a total pumping chamber volume based on the pressure and temperature measurements taken during the empty volume state.

Aspect 34. The system according to Aspect 30 and 33, wherein the controller is configured to calculate a stroke volume according to the difference between the total pumping chamber volume and the pump side pumping chamber volume.

Aspect 35. The system according to any one of Aspects 29 to 34, wherein the temperature measurements are based on an average of temperature measurements from a plurality of temperature sensors and an ambient temperature.

Aspect 36. The system according to Aspect 35, wherein the plurality of temperature sensors are associated with the common volume, the pumping chamber, and a positive pressure tank and a negative pressure tank coupled to the common volume.

Aspect 37. The system according to Aspect 35 or 36, wherein the controller is configured to calculate the temperature measurements as mass-weighted average temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature.

Aspect 38. The system according to Aspect 37, wherein the controller is configured to calculate the administrative set temperature according to:

$$T_S = T_B + \beta(T_M - T_B),$$

where Ts is the administrative set temperature;
$T_A$ is the ambient temperature;
$\beta$ is a parameter that accounts for pre-heating of the set side pumping chamber volume by pump side pumping chamber air; and
$T_M$ is the average of temperature measurements of the plurality of temperature sensors.

Aspect 39. The system according to any one of Aspects 35 to 38, wherein the average of temperature measurements of the plurality of temperature sensors is taken of temperature measurements of a subset of the plurality of temperature sensors.

Aspect 40. The system according to any one of Aspects 35 to 39, wherein each of the plurality of temperature sensors comprise a temperature sensor associated with a pressure sensor.

Aspect 41. The system according to any one of Aspects 29 to 34, wherein the controller is configured to isolate the filled pumping chamber from the fluid source and a recipient before applying the negative gauge pressure to the diaphragm with the pumping chamber filled with the fluid, and before applying the positive gauge pressure to the diaphragm with the pumping chamber filled with the fluid.

Aspect 42. The system according to Aspect 41, further comprising an inlet valve disposed between the pumping chamber and the fluid source and an outlet valve disposed between the pumping chamber and the recipient, and the controller is configured to isolate the pumping chamber by closing the inlet valve and the outlet valve.

**Claims**

1. A method comprising:

   applying a negative gauge pressure from a common volume to a diaphragm in a pumping chamber to fill the pumping chamber with a fluid from a fluid source;
   applying the negative gauge pressure to the diaphragm with the pumping chamber filled with the fluid to define a negative full-volume state, and measuring temperature and pressure of the common volume and the pumping chamber during the negative full-volume state;
   applying a positive gauge pressure to the diaphragm with the pumping chamber filled with the fluid to define a positive full-volume state, and measuring the temperature and pressure of the common volume and the pumping chamber during the positive full-volume state;
   applying the positive gauge pressure to the diaphragm with the pumping chamber empty to define an empty-volume state, and measuring the temperature and
   pressure of the common volume and the pumping chamber during the empty-volume state; and
   calculating a volume of fluid in the pumping chamber when the chamber is not full or empty based on the pressure and temperature measurements taken during the negative full-volume state, the positive full-volume state, and the empty-volume state.

2. The method according to claim 1, further comprising:

   calculating a pump side pumping chamber volume based on the pressure and temperature measurements taken during the negative full-volume state;

calculating a total volume of air in the pumping chamber on air side and fluid side based on the pressure and temperature measurements taken during the positive full volume state; and

calculating a difference between the pump side pumping chamber volume and the total volume of air in the pumping chamber to calculate a volume of air on the fluid side of the pumping chamber.

3. The method according to claim 2, further comprising:

calculating a total pumping chamber volume based on the pressure and temperature measurements taken during the empty volume state; and

calculating a stroke volume according to the difference between the total pumping chamber volume and the pump side pumping chamber volume.

4. The method according to any one of claims 1 to 3, wherein the temperature measurements are based on an average of temperature measurements from a plurality of temperature sensors and an ambient temperature.

5. The method according to claim 4, wherein:

the temperature measurements are calculated as mass-weighted average temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature; and

the administrative set temperature is calculated according to:

$$T_S = T_A + \beta(T_M - T_A),$$

where Ts is the administrative set temperature;

$T_A$ is the ambient temperature;

$\beta$ is a parameter that accounts for pre-heating of the set side pumping chamber volume by pump side pumping chamber air; and

$T_M$ is the average of temperature measurements of the plurality of temperature sensors.

6. The method according to claim 4 or 5, wherein the average of temperature measurements of the plurality of temperature sensors is taken of temperature measurements of a subset of the plurality of temperature sensors.

7. The method according to any one of claims 1 to 3, further comprising:

isolating the filled pumping chamber from the fluid source and a recipient before applying the negative gauge pressure to the diaphragm with the pumping chamber filled with the fluid, and before applying the positive gauge pressure to the diaphragm with the pumping chamber filled with the fluid,

wherein an inlet valve is disposed between the pumping chamber and the fluid source, an outlet valve is disposed between the pumping chamber and the recipient, and isolating the pumping chamber comprises closing the inlet valve and the outlet valve.

8. An infusion system comprising:

a common volume;

a pumping chamber comprising a diaphragm, a fluid inlet coupled to a fluid source, and a fluid outlet, the pumping chamber coupled to the common volume;

a pressure sensor and a temperature sensor associated with each of the common volume and the pumping chamber;

a controller coupled to the pressure sensors and temperature sensors, the controller configured to:

apply a negative gauge pressure from the common volume to the diaphragm in the pumping chamber to fill the pumping chamber with a fluid from the fluid source;

apply the negative gauge pressure to the diaphragm with the pumping chamber filled with the fluid to define a negative full-volume state, and measure temperature and pressure of the common volume and the pumping chamber during the negative full-volume state;

apply a positive gauge pressure to the diaphragm with the pumping chamber filled with the fluid to define a positive full-volume state, and measure the temperature and pressure of the common volume and the pumping chamber during the positive full-volume state;

apply the positive gauge pressure to the diaphragm with the pumping chamber empty to define an empty-volume state, and measure the temperature and pressure of the common volume and the pumping chamber during the empty-volume state; and

calculate a volume of fluid in the pumping chamber when the chamber is not full or empty based on the pressure and temperature measurements taken during the negative full-volume state, the positive full-volume state, and the empty-volume state.

9. The system according to claim 8, wherein the controller is configured to calculate a pump side pumping chamber volume based on the pressure and temperature measurements taken during the negative full-volume state, to calculate a total amount of air in the pumping chamber on air side and fluid side based on the pressure and temperature measurements taken during the positive full volume state, and to calculate a difference between the pump side pumping chamber volume and the total volume of air in the pumping chamber to calculate a volume of air on the fluid side of the pumping chamber.

10. The system according to claim 9, wherein the controller is configured to calculate a total pumping chamber volume based on the pressure and temperature measurements taken during the empty volume state, and to calculate a stroke volume according to the difference between the total pumping chamber volume and the pump side pumping chamber volume.

11. The system according to any one of claims 8 to 10, wherein the temperature measurements are based on an average of temperature measurements from a plurality of temperature sensors and an ambient temperature.

12. The system according to claim 11, wherein the plurality of temperature sensors are associated with the common volume, the pumping chamber, and a positive pressure tank and a negative pressure tank coupled to the common volume.

13. The system according to claim 11 or 12, wherein the controller is configured:

to calculate the temperature measurements as mass-weighted average temperatures, and a pumping chamber temperature is based on a constant volume region within the pumping chamber at the average of temperature measurements of the plurality of temperature sensors and a remaining volume region of an air side of the pumping chamber at an administrative set temperature that is based on the average of the temperature measurements of the plurality of temperature sensors and the ambient temperature; and
to calculate the administrative set temperature according to:

$$T_S = T_A + \beta(T_M - T_A),$$

where Ts is the administrative set temperature;
$T_A$ is the ambient temperature;
$\beta$ is a parameter that accounts for pre-heating of the set side pumping chamber volume by pump side pumping chamber air; and
$T_M$ is the average of temperature measurements of the plurality of temperature sensors.

14. The system according to any one of claims 11 to 13, wherein:

the average of temperature measurements of the plurality of temperature sensors is taken of temperature measurements of a subset of the plurality of temperature sensors; and
each of the plurality of temperature sensors comprise a temperature sensor associated with a pressure sensor.

15. The system according to any one of claims 8 to 10, further comprising an inlet valve disposed between the pumping chamber and the fluid source and an outlet valve disposed between the pumping chamber and the recipient,

wherein the controller is configured to isolate the filled pumping chamber from the fluid source and a recipient before applying the negative gauge pressure to the diaphragm with the pumping chamber filled with the fluid, and before applying the positive gauge pressure to the diaphragm with the pumping chamber filled with the fluid, and

wherein the controller is configured to isolate the pumping chamber by closing the inlet valve and the outlet valve.

**FIG. 1**

**FIG. 2**

**FIG. 3**

**FIG. 4**

**FIG. 5**

**FIG. 6**

**FIG. 7**

APPLY PRESSURE TO
EMPTY PUMPING
CHAMBER

↓

MEASURE
TEMPERATURE AND
PRESSURE

↓

CALCULATE VOLUME OF
FLUID IN PUMPING
CHAMBER

**FIG. 8**

APPLY NEGATIVE
GAUGE PRESSURE TO
FILL PUMPING CHAMBER

↓

APPLY NEGATIVE GAUGE
PRESSURE TO FILLED
PUMPING CHAMBER,
AND MEASURE
TEMPERATURE AND
PRESSURE

↓

APPLY POSITIVE GAUGE
PRESSURE TO FILLED
PUMPING CHAMBER,
AND MEASURE
TEMPERATURE AND
PRESSURE

↓

APPLY POSITIVE GAUGE
PRESSURE TO EMPTY
PUMPING CHAMBER,
AND MEASURE
TEMPERATURE AND
PRESSURE

↓

CALCULATE VOLUME OF
FLUID IN PUMPING
CHAMBER IN
INTERMEDIATE STATE

**FIG. 9**

Europäisches
Patentamt

European
Patent Office

Office européen
des brevets

**EUROPEAN SEARCH REPORT**

Application Number

EP 24 16 1303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/343936 A1 (GRAY LARRY B [US]) 26 December 2013 (2013-12-26) | 1-3, 7-10,15 | INV. A61M5/142 |
| A | * paragraph [0085] * * paragraphs [0091] - [0146] * * figures 1-9 * | 4-6, 11-14 | A61M5/168 A61M5/172 |
| A | US 2014/216560 A1 (AMBROSINA JESSE E [US] ET AL) 7 August 2014 (2014-08-07) * the whole document * | 1-15 | |

TECHNICAL FIELDS
SEARCHED      (IPC)

A61M

The present search report has been drawn up for all claims

| Place of search | Date of completion of the search | Examiner |
|---|---|---|
| Munich | 11 July 2024 | Kollar, Julien Felix |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 16 1303

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2024

| Patent document cited in search report | Publication date | Patent family member(s) | Publication date |
|---|---|---|---|
| US 2013343936 A1 | 26-12-2013 | US 6877713 B1 | 12-04-2005 |
| | | US 2005069425 A1 | 31-03-2005 |
| | | US 2009202367 A1 | 13-08-2009 |
| | | US 2010296953 A1 | 25-11-2010 |
| | | US 2013343936 A1 | 26-12-2013 |
| | | US 2014194820 A1 | 10-07-2014 |
| | | US 2014260551 A1 | 18-09-2014 |
| | | US 2014260556 A1 | 18-09-2014 |
| | | US 2014276428 A1 | 18-09-2014 |
| | | US 2014286794 A1 | 25-09-2014 |
| | | US 2015359956 A1 | 17-12-2015 |
| | | US 2017356435 A1 | 14-12-2017 |
| US 2014216560 A1 | 07-08-2014 | AU 2014215580 A1 | 13-08-2015 |
| | | AU 2017204557 A1 | 27-07-2017 |
| | | CA 2899727 A1 | 14-08-2014 |
| | | CN 105163776 A | 16-12-2015 |
| | | CN 105288772 A | 03-02-2016 |
| | | CN 110115788 A | 13-08-2019 |
| | | EP 2953665 A1 | 16-12-2015 |
| | | HK 1215406 A1 | 26-08-2016 |
| | | HK 1221183 A1 | 26-05-2017 |
| | | JP 6356154 B2 | 11-07-2018 |
| | | JP 2016512972 A | 12-05-2016 |
| | | US 2014216560 A1 | 07-08-2014 |
| | | US 2014309617 A1 | 16-10-2014 |
| | | WO 2014123816 A1 | 14-08-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10156231 B
- US 9433734 B
- US 9849231 B
- US 9616172 B
- US 10010686 B
- US 10444770 B
- US 10881785 B
- US 11285262 B